(19) **Europäisches Patentamt / European Patent Office / Office européen des brevets**

(11) **EP 2 893 027 B1**

(12) **EUROPEAN PATENT SPECIFICATION**

(45) Date of publication and mention
of the grant of the patent:
**19.10.2016 Bulletin 2016/42**

(21) Application number: **13758850.5**

(22) Date of filing: **04.09.2013**

(51) Int Cl.:
**G01N 27/327** (2006.01)   **C12Q 1/00** (2006.01)

(86) International application number:
**PCT/EP2013/068238**

(87) International publication number:
**WO 2014/037372 (13.03.2014 Gazette 2014/11)**

(54) **IMPROVED MATRIX STABILITY COMPOSITIONS AND METHODS**

ZUSAMMENSETZUNGEN MIT VERBESSERTER MATRIXSTABILITÄT UND VERFAHREN DAFÜR

COMPOSITIONS ET PROCÉDÉS DE STABILITÉ AMÉLIORÉE DE MATRICE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.09.2012 US 201261697535 P**

(43) Date of publication of application:
**15.07.2015 Bulletin 2015/29**

(73) Proprietors:
• **Roche Diabetes Care GmbH**
**68305 Mannheim (DE)**
Designated Contracting States:
**DE**
• **F.Hoffmann-La Roche AG**
**4070 Basel (CH)**
Designated Contracting States:
**AL AT BE BG CH CY CZ DK EE ES FI FR GB GR HR HU IE IS IT LI LT LU LV MC MK MT NL NO PL PT RO RS SE SI SK SM TR**

(72) Inventors:
• **DUVALL, Stacy**
**Indianapolis, Indiana 46256 (US)**
• **KACHEL, Sibylle**
**76131 Karlsruhe (DE)**
• **LICA, Georgeta Cami**
**Indianapolis, Indiana 46202 (US)**
• **SCHABEL, Wilhelm**
**76229 Karlsruhe (DE)**
• **SCHARFER, Philip**
**76344 Eggenstein-Leopoldshafen (DE)**

(74) Representative: **Poredda, Andreas et al**
**Roche Diagnostics GmbH**
**Patentabteilung**
**Sandhofer Strasse 116**
**68305 Mannheim (DE)**

(56) References cited:
**EP-A2- 1 174 716        WO-A1-01/08784
WO-A1-97/27483        WO-A1-02/083273
WO-A1-2008/130680    WO-A2-02/50609
GB-A- 2 499 838        US-A1- 2008 031 778**

**Description**

The present invention relates to a test element for determining the amount of an analyte in a sample fluid and a method for improving the stability of a biosensor test element.

As the number of patients suffering from diabetes and similar medical conditions increases, self-monitoring of blood glucose wherein the patient monitors his or her blood glucose levels has become a common practice. The purpose of monitoring the blood glucose level is to determine the concentration level and then to take corrective action, based upon whether the level is too high or too low, to bring the level back within a normal range. The failure to take corrective action can have serious medical implications, so glucose monitoring is a fact of everyday life for diabetic individuals. Failure to test blood glucose levels properly and on a regular basis can result in serious diabetes-related complications, including cardiovascular disease, kidney disease, nerve damage and blindness.

Biosensors are available to permit an individual to test the glucose level in a small sample of blood. Common meter designs use a disposable test element which, in combination with the meter, measures the amount of glucose in the blood sample electrochemically or optically. The information is typically displayed as a blood glucose value and perhaps the time and date the measurement was performed. This information is in most cases sufficient to allow diabetics to adjust their dietary intake and/or insulin dosage, and in the case of low glucose values may indicate the need for intake of sugar to avoid hypoglycemia.

The electrochemical measurement of an analyte such as glucose may be achieved by dosing a sensor with a sample containing the analyte (for example, glucose in an aqueous blood sample) to initiate a chain of reactions such as the chain shown below for glucose. By applying a potential difference between the working and counter electrodes, the reduced form of the mediator is converted to the oxidized form. The current associated with this reaction is proportional with the mass of reduced mediator, and consequently with the glucose concentration.

$$\text{Glucose} \rightleftarrows \text{Enzyme-Red} \rightleftarrows \text{Med1}_{red}$$
$$\text{Gluconolactone} \qquad \text{Enzyme-Ox} \qquad \text{Med1}_{ox}$$

Exposure of biosensor test elements (such as glucose test strips) to high humidity conditions may cause the mediator to degrade to the reduced form or to other products that are electrochemically active in the same potential range. Additionally, high humidity may cause the enzyme to degrade to a reduced form, and the reduced enzyme may react with the mediator, producing mediator in its reduced form.

The accumulation of electroactive degradation products at the electrode surface and corresponding conversion under applied potential will result in current generation even in the absence of a substrate (blank current). This current can add to the current generated by the glucose reaction resulting in a positively biased result or increased strip failure rate.

The components of a test strip reagent matrix are often selected to dissolve quickly in an aqueous blood sample, thus providing a fast reaction time and a quick display of the result for the user. However this provides the undesired consequence of making the matrix susceptible to degradation by environmental humidity, thus compromising the shelf stability of disposable test strips.

Accordingly, a method for decreasing the test element's sensitivity to environmental conditions is desired. EP 1174716 describes a biosensor including a second insulating substrate situated on a first substrate, tracks situated between the substrates, a testing reagent, a third substrate situated over the reagent on a portion of the second substrate, and a cover that extends over the third substrate. A desiccant may be permanently applied to the cover or the third substrate. US 2008/0031778 also describes a test element, wherein a desiccant is contained in the test element in a preferred embodiment. WO 02/50609 relates to electrochemical test strip cards that include an integral desiccant. WO 02/083273 relates to a desiccant composition comprising a deliquescent salt and modified starch. While test elements can be packaged and distributed in containers configured to prevent undesired ingress of moisture from the atmosphere or other sources, and while test elements can be packaged in containers with desiccant materials incorporated within the container (such as in the lid or cap of the container) or in an insert that is left in the container with the test elements, known methods have not been effective for eliminating the risks of sensor degradation.

A need therefore exists for compositions and methods for improving the stability of biosensor tests systems, and more particularly of glucose test strips. The present invention addresses that need.

The present invention is as defined in the claims. In one aspect of the present invention there is provided a test element for determining the amount of an analyte in a sample fluid. The test element comprises a working electrode and a counter electrode, and a reagent matrix extending between the working electrode and the counter electrode and configured for an electrode reaction. The reagent matrix is provided with a deliquescent material, and the deliquescent material is selected and provided in an amount effective to protect one or more of the active ingredients from humidity in the atmosphere.

Herein described is also a reagent matrix for a blood glucose biosensor, comprising a mediator, an enzyme, and a deliquescent material selected and provided in an amount effective to preferentially and/or preemptively absorb water from the air when compared to the water absorption properties and rates of the other components of the reagent matrix, thus protecting the other components from degradation by water when the relative humidity exceeds the deliquescence point of the deliquescent material. The deliquescent material may be selected to preferentially absorb water when the environmental relative humidity exceeds any predetermined level, such as 40%, or 50%, or 60%, or 70%, or 75%, or 80%, etc., as desired.

In another aspect of the present invention there is provided a method for improving the stability of a biosensor test element with respect to degradation of reagent components by humidity in the air, wherein the test element comprises a base/supporting substrate, working and counter electrodes, and a reagent matrix extending between the working electrode and the counter electrode and configured for an electrode reaction. The inventive method comprises providing in the reagent matrix a deliquescent material selected and in an amount effective to decrease the sorption of water from the air by other components of the reagent matrix.

Herein described is also a method for improving the stability of a dry-film reagent matrix of an electrochemical biosensor. This method may comprise providing in the reagent matrix a deliquescent material selected and in an amount effective to decrease the sorption of water from the air by other components of the reagent matrix.

The deliquescent material is selected to be a material having a deliquescence point between 50% relative humidity and 80% relative humidity. In some embodiments the deliquescence point is preferably between 70% relative humidity and 80% relative humidity, while in other embodiments the deliquescent material has a deliquescence point at 75% relative humidity.

The test element may be a glucose meter test strip having a glucose reagent matrix thereon, with the test strip reagent matrix including an enzyme system and a mediator. The enzyme system may comprise glucose dehydrogenase (GDH) enzyme and/or flavin adenine dinucleotide (FAD) cofactor. The mediator may comprise a nitrosoaniline mediator. A film former comprising polyvinylpyrrolidone (PVP) may also be included.

The deliquescent material may comprise one or more members selected from the group consisting of sodium chloride, calcium chloride, magnesium chloride, zinc chloride, potassium carbonate, potassium phosphate, carnallite, ferric ammonium citrate, potassium hydroxide and sodium hydroxide. The deliquescent material may be provided in an amount effective to absorb water from the atmosphere at a rate that is faster than the rate at which the other components of the reagent matrix absorb water from the atmosphere. Similarly, the deliquescent material may be provided in an amount effective to absorb water from the atmosphere at a rate that is faster than the rate at which the other components of the reagent matrix absorb water from the atmosphere when the relative humidity in the atmosphere exceeds about 75%. The deliquescent material may alternatively or additionally be selected and provided in an amount effective to preferentially absorb water from the atmosphere when compared to the water absorption properties of the other reagent components, thus protecting the other components of the reagent matrix from absorbing water from the atmosphere when the relative humidity exceeds the deliquescence point of the deliquescent material.

FIG. 1 shows a graph of the blank current (nA) over time for strips having varying amounts of NaCl added to the reagent mixture. The strips were dosed with buffer solution and exposed to high heat and humidity conditions (30°C/80%RH).

FIG. 2 shows a graph of the blank current (nA) over time for strips having varying amounts of NaCl added to the reagent mixture. The strips were dosed with unspiked blood and exposed to high heat and humidity conditions (30°C/80%RH).

FIG. 3 shows a graph of the fraction of mediator remaining on the strip over time for strips having varying amounts of NaCl added to the reagent mixture. The strips were exposed to high heat and humidity conditions (30°C/80%RH).

FIG. 4 is a graph of sorption data for PVP according to test measurements (red diamonds) and reported literature values (other symbols).

FIG. 5 is a graph of sorption data for NaCl according to test measurements (red diamonds) and reported literature values (other symbols), as well as the deliquescence point (green line) and the crystallization point (blue line).

FIG. 6 is a graph of sorption isotherms for PVP, NaCl, and mixtures of the two components.

FIG. 7 is a graph of the blank currents observed when dry film reagents having various additives are protected by deliquescent materials, as provided by certain aspects of the present invention.

For the purpose of promoting an understanding of the principles of the invention, reference will now be made to certain embodiments and specific language will be used to describe the same. It will nevertheless be understood that no limitation of the scope of the invention is thereby intended. Any alterations and further modifications in the described embodiments, and any further applications of the principles of the invention as described herein, are contemplated as would normally occur to one skilled in the art to which the invention relates. In particular, although the most preferred embodiments of the invention relate to blood glucose test device and measurement methods, it is contemplated that the invention can be used with devices for measuring other analytes and other sample types. Such alternative embodiments require certain adaptations to the embodiments discussed herein that would be obvious to those skilled in the art.

Glucose biosensors typically use a reagent matrix comprising an enzyme/cofactor system and a mediator to test for glucose concentration in a blood sample. When glucose sensor test strips are exposed to high humidity for extended periods of time, the strips may lose their functionality or become unstable. The present invention addresses that problem by formulating the test strip reagent matrix to include a deliquescent material that absorbs water from the atmosphere at a selected humidity level, thereby preventing the water from degrading the reagent matrix components. The deliquescent material is selected to protect the reagent components when exposed to a defined relative humidity, such as when the relative humidity in the atmosphere exceeds about 75%. The deliquescent material is preferably a salt, and is most preferably sodium chloride. Other known deliquescent materials include calcium chloride, magnesium chloride, zinc chloride, potassium carbonate, potassium phosphate, carnallite, ferric ammonium citrate, potassium hydroxide and sodium hydroxide.

The reagent matrix stabilization materials and methods of the present invention have particular utility when used with test elements (such as a glucose test strip) having dry-film reagent systems that may degrade if exposed to excessive humidity, such as a relative humidity of 75%, or even 70% or less. For example, commonly-used glucose reaction mediators may degrade when exposed to humidity (water) in the air, producing a reduced form or other products which are electrochemically active and can result in a blank current in the test meter. Similarly, an enzyme may degrade to a reduced form, then reducing the mediator and presenting that problem by this route. The issue is exacerbated by the fact that the reagent matrix needs a degree of hydrophilicity in order to provide quick wetting of the reagent (and thus, a quick test readout). This issue is specifically presented, for example, when PVP-containing materials are used as a film former for a glucose reagent system. PVP adsorbs moisture from the air - bringing it into contact with the reagent system.

In one aspect of the present invention the problem noted above is addressed by adding a component to the reagent matrix that causes the matrix as a whole to adsorb less water. While the invention is illustrated in this disclosure by adding a deliquescent material such as NaCl to a glucose reagent system including FADGDH and nitrosoaniline, the invention when construed broadly is intended to include other reagent systems and other deliquescent materials.

In the most preferred embodiments of the present invention the biosensor is a glucose test strip. Such test strips typically have working and counter electrodes, and are provided with a reagent matrix to electrochemically analyze a test strip reagent matrix. However, other devices and processes of this kind are within the scope of the present disclosure, which may utilize one or more alternative test elements. These test elements are known and available in different forms, to which the present invention as a whole is applicable. For example, test elements in the form of test strips, test tapes, test disks, foldable test elements (for example, according to the Leporello principle) and other forms as are known to persons skilled in the art. Hereinafter, while the invention will be described substantially with reference to test strips, it is to be appreciated that other embodiments are also possible and are intended to be within the scope of the disclosed invention.

The test element typically comprises at least one test field having a test field surface. A test field is understood to mean a two- or three-dimensional region of the test element, which region is usable in principle for the detection of the analyte, which can be carried out qualitatively and/or quantitatively. In some embodiments the test field may be a dry test field, and may comprises at least one detection reagent which is selected to carry out a detectable reaction in the presence of the analyte.

In certain preferred embodiments of the present invention a test strip reagent matrix comprises an enzyme system and a mediator. The enzyme system may comprises glucose dehydrogenase enzyme (GDH) and/or flavin adenine dinucleotide cofactor (FAD), and the mediator may comprises a nitrosoaniline mediator. The reagent matrix may also include a film former, which may comprise polyvinylpyrrolidone (PVP). Examples of other glucose-specific enzymatic detection reagents include, but are not limited to, deoxy reductases (e.g., GlucDOR/PQQ), dehydrogenases, oxidases, or similar enzymes, for example glucose oxidase (GOD) or glucose dehydrogenase.

A mediator may also be included in the reagent mixture. The mediator may be any chemical species (generally electroactive), which can participate in a reaction scheme involving an enzyme, an analyte, and optionally a cofactor (and reaction products thereof), to produce a detectable electroactive reaction product. Typically, participation of the mediator in this reaction involves a change in its oxidation state (e.g., a reduction), upon interaction with any one of the analyte, the enzyme, or a cofactor, or a species that is a reaction product of one of these (e.g., a cofactor reacted to a different oxidation state). A variety of mediators exhibit suitable electrochemical behavior. A mediator can preferably also be stable in its oxidized form; may optionally exhibit reversible redox electrochemistry; can preferably exhibit good solubility in aqueous solutions; and preferably reacts rapidly to produce an electroactive reaction product. Examples of suitable mediators include benzoquinone, meldola blue, other transition metal complexes, potassium ferricyanide, and nitrosoanilines, see U.S. Patent No. 5,286,362.

In addition to the enzymes, mediators, and other detection reagent components, the test field may further include carrier substances, auxiliary substances, pigments, fillers, buffer substances, etc. Hereinafter, no distinction is made between further substances which are likewise involved in the reaction for detecting the analyte, and the actual detection reagent.

It is to be appreciated that the chemistry of the reaction scheme of any chosen electrochemical detection method can be chosen in light of various chemical factors relating to the system, including the identity of the analyte and of the sample substance. Even then, for a given analyte or substance, various different reactive components may be useful in terms of a catalyst (often, a variety of enzymes will be useful), co-reactants (e.g., a variety of mediators may be useful), and cofactors (if needed, a variety may be useful). Many such reaction schemes and their reactive components and reaction products are known, and examples of a few different enzymes include those listed in Table 1.

TABLE 1

| Analyte | Enzymes | Redox Mediator (Oxidized Form) | Additional Mediator |
|---|---|---|---|
| Glucose | Glucose dehydrogenase and Diaphorase | Ferricyanide, osmium (III)-(bipyridyl)-2-imidazolyl-chloride, Meldola blue, $[Ru(NH_3)_5MeIm] Cl_3 [OS(III)(NH_3)_5pyz]_2(SO_4)_3$, NITROSO aniline derivatives | |
| Glucose | Glucose oxidase | (see above) | |
| Cholesterol | Cholesterol esterase and Cholesterol oxidase | (see glucose) | 2,6-Dimethyl-1,4-Benzoquinone, 2, 5-Dichloro-1,4-benzoquinone, or phenazine ethosulfate |
| HDL Cholesterol | Cholesterol esterase and Cholesterol oxidase | (see glucose) | 2,6-Dimethyl-1,4-Benzoquinone, 2, 5-Dichloro-1,4-benzoquinone, or phenazine ethosulfate |
| Triglycerides | Lipoprotein lipase, Glycerol kinase, Glycerol-3-phosphate oxidase | (see glucose) | Phenazine methosulfate, phenazine ethosulfate. |
| Triglycerides | Lipoprotein lipase, Glycerol kinase, Glycerol-3-phosphate dehydrogenase and Diaphorase | (see glucose) | Phenazine methosultate, phenazine ethosulfate. |
| Lactate | Lactate oxidase | (see glucose) | 2, 5-Dichloro-1,4-benzoquinone |
| Lactate | Lactate dehydrogenase and Diaphorase | (see glucose) | |
| Lactate Dehydrogenase | Diaphorase | (see glucose) | |
| Pyruvate | Pyruvate oxidase | (see glucose) | |
| Alcohol | Alcohol oxidase | (see glucose) | |
| Alcohol | Alcohol dehydrogenase | (see glucose) | |
| | and Diaphorase | | |
| Uric acid | Uricase | (see glucose) | |
| 3-Hydroxybutric acid (ketone bodies) | 3-Hydroxybutyrate dehydrogenase and Diaphorase | (see glucose) | |

To further describe an example of an oxidation/reduction reaction scheme that is known to be useful for detecting glucose in human blood, a sample containing glucose can react with an enzyme (e.g., Glucose-Dye-Oxidoreductase (Gluc-Dor)) and optionally a cofactor, (e.g., pyrrolo-quinoline-quinone), in the presence a redox mediator (e.g., benzoquinone, ferricyanide, or nitrosoaniline derivatives), to produce the oxidized form of the analyte, gluconolactone, and the reduced form of the redox mediator. *See,* U.S. Patent No. 5,128,015. Other examples of reaction schemes are known, and are typically used in methods designed to detect a specific analyte, *e.g.,* cholesterol, urea, etc.

Regardless of the reagent matrix used, a deliquescent material is added to the reagent matrix to protect components of the reagent from degradation by water. Preferred deliquescent materials include sodium chloride, calcium chloride, magnesium chloride, magnesium sulfate, zinc chloride, potassium carbonate, potassium phosphate, carnallite, ferric ammonium citrate, potassium hydroxide and sodium hydroxide, with salts such as sodium chloride being most preferred.

As used herein, the term "deliquescence" generally refers to a phase transition from solid to solution that occurs when the vapor pressure of a saturated aqueous solution of a substance is less than the vapor pressure of water in ambient air. When water vapor is collected by the pure solid compound, a mixture of the solid and its saturated solution or an aqueous solution of the compound forms until the substance is dissolved and is in equilibrium with its environment. The relative humidity at which deliquescence occurs is a property of the specific substance. At this point the vapor pressure of water over the aqueous solution will equal the partial pressure of water in the atmosphere in contact with it. Accordingly, a salt particle will deliquesce in the atmosphere when the relative humidity surpasses the deliquescence point of the material. The term "deliquescent material" generally refers to materials having deliquescent properties, and may refer to materials, such as salts, that have a strong affinity for moisture and that will absorb relatively large amounts of water from the atmosphere if exposed to it, thus protecting other materials from absorbing water.

As indicated above, the deliquescent material may be selected to be a material having a deliquescence point that protects the other reagent components when exposed to environmental humidity at a pre-determined level. Accordingly, in one aspect of the present invention a user identifies a particular environmental stress to be avoided, such as the environmental stress of a 75% relative humidity, and includes in the reagent mixture a deliquescent material selected to have a deliquescence point at slightly below that level. For example, the deliquescence point of NaCl is near a relative humidity (RH) of 75%, so the addition of NaCl is particularly effective for use with test elements that may be exposed to a relative humidity that may exceed 75%. When the relative humidity exceeds that point, the sodium chloride crystals absorb water faster than the other reagent materials, thus preempting absorption by the other components and protecting the other reagent materials from water degradation.

The amount of deliquescent material added to the reagent matrix will depend on the material being used, the other components in the reagent matrix, and the environmental conditions likely to be faced. In general, the deliquescent material will be provided in the reagent matrix in an amount effective to allow the deliquescent material to preemptively take on the water that would otherwise be taken on by other reagent components, and thus to prevent the other reagent components from deteriorating. In other words, the deliquescent material is preferably provided in an amount effective to absorb water from the atmosphere at a rate that is faster than the rate at which the other components of the reagent matrix will absorb water from the atmosphere, thus preventing the other components from absorbing water and degrading.

In some embodiments the deliquescent material may be a combination of two or more deliquescent materials. For example, a mixture of salts such as NaCl, $MgSO_4$ and $CaCl_2$ may be used.

Whether used individually or in combination, the deliquescent material(s) may be added to the reagent matrix in amounts effective to provide from about 1% to about 10% by weight percent deliquescent material in the dry film reagent, with amounts between about 2% and about 8% being more preferred. In one embodiment, the deliquescent material is provided in the dry film reagent matrix in an amount between about 3% and about 7% by weight, with amounts between about 3% and about 6% being more preferred. In one embodiment, the deliquescent material is included in the reagent material in an amount effective to provide at least about 2% deliquescent material in the dry film reagent matrix, while in another embodiment the deliquescent material is included in the reagent material in an amount effective to provide at least about 3% deliquescent material in the dry film reagent matrix.. In another embodiment, the deliquescent material may be added to the reagent material in amounts effective to provide no more than about 10% deliquescent material in the dry film reagent matrix.

In one preferred embodiment the deliquescent material was provided in an amount effective to provide about 3.0% NaCl in the dry film reagent. In another preferred embodiment the deliquescent material was provided in an amount effective to provide about 5.8% NaCl in the dry film reagent. In a third preferred embodiment the deliquescent material was provided in an amount that provides about 6.5% $MgSO_4$ in the dry film reagent.

As indicated above, the principles of the invention described herein find particular utility in stabilizing reagents used in a test element for detecting at least one analyte in a sample, such as the detection of at least one metabolite in a body fluid, and particularly the detection of glucose in a blood sample. Accordingly, certain aspects and principles of the present invention are illustrated herein by describing one particularly preferred embodiment, namely, an embodiment in which NaCl is used as a deliquescent material in a reagent mixture containing PVP or a PVP-containing material. By understanding the water sorption behavior of PVP and sodium chloride, the mechanism for improved mediator stability in films containing sodium chloride can be better understood. This understanding facilitates the design of biosensor matrices when applied to the materials discussed above as well as new matrix components.

EXAMPLE 1

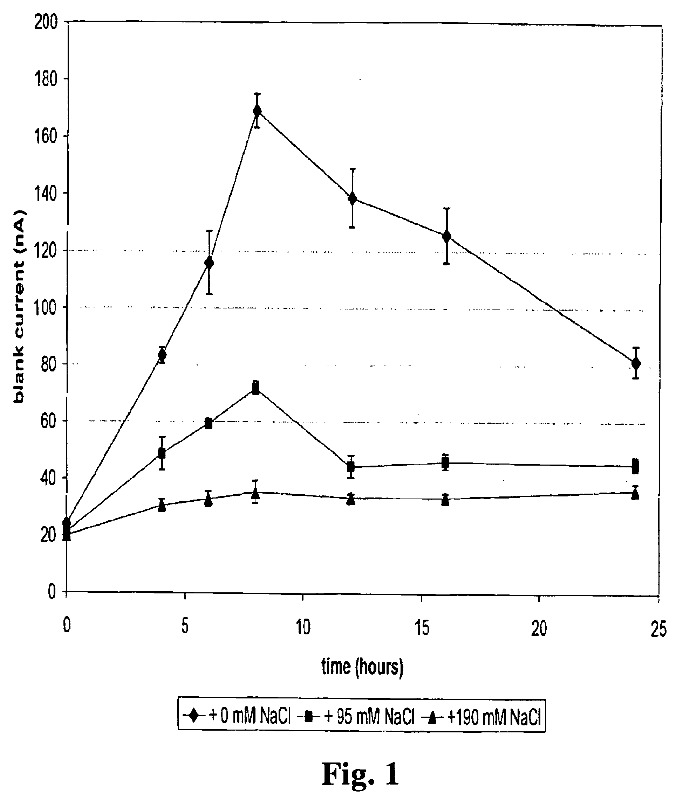

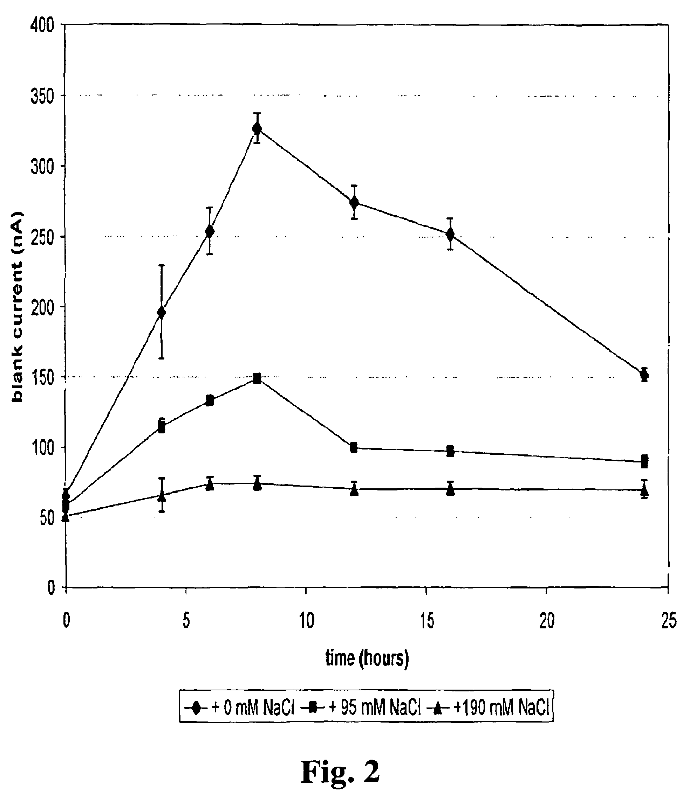

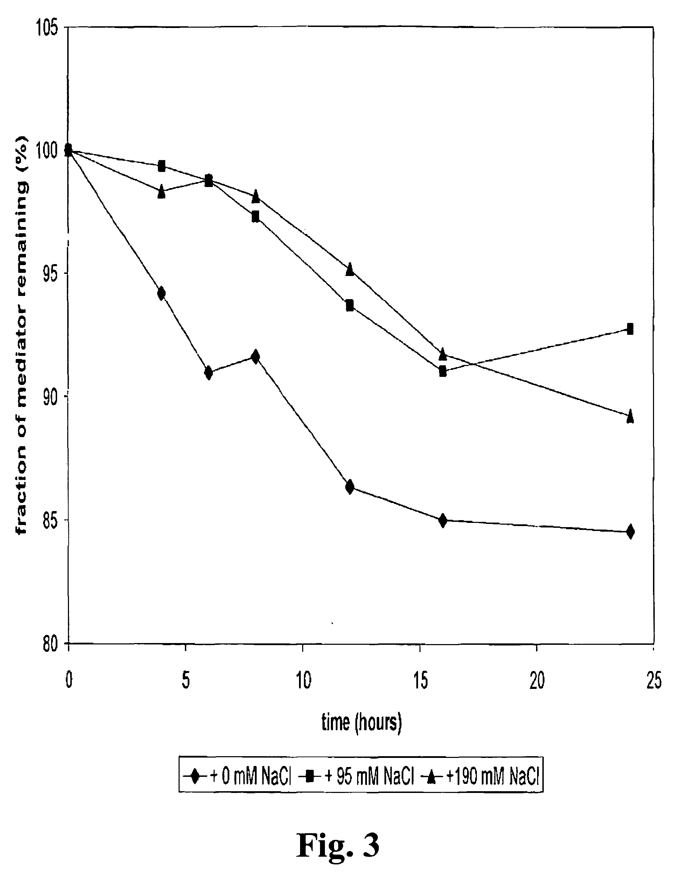

Sodium chloride was added to the reagent to increase the ionic strength of the reagent. The blank current in both aqueous solution and in unspiked blood was significantly decreased in formulations that contained additional sodium chloride when compared to the formulation that did not contain additional sodium chloride (see Figures 1 and 2). Mediator stability as measured independently by HPLC was better in strips with the additional sodium chloride, as shown in Figure 3. The result is increased stability of the reagent matrix. As shown in FIG. 3, increasing the amount of a deliquescent material such as NaCl in a reagent matrix increases the fraction of mediator remaining on the strip after exposure to high humidity.

EXAMPLE 2

Since exposure to high heat and humidity is known to cause mediator stability failure in glucose biosensors, the sorption behavior of PVP, NaCl, and mixtures of PVP and NaCl were studied. Such studies help characterize the sorption properties of the reagent matrix without studying the complete reagent matrix and are useful as a predictive tool for designing reagent matrix films with desirable water sorption characteristics.

The sorption isotherms of PVP and NaCl were measured and modeled, then compared with the measured sorption isotherms of mixtures of PVP and NaCl. Where appropriate, the measured data was modeled with the Flory-Huggins equation:

$$\ln a_w = \ln \varphi_w + \varphi_P + \chi_{wP} \cdot \varphi^2_P,$$

Where $a_w$ is the water activity in the gas phase (relative humidity), $\varphi$ is the volume fraction of water (w) and polymer (P), and $\chi_{wP}$ is the interaction parameter between the water and polymer.

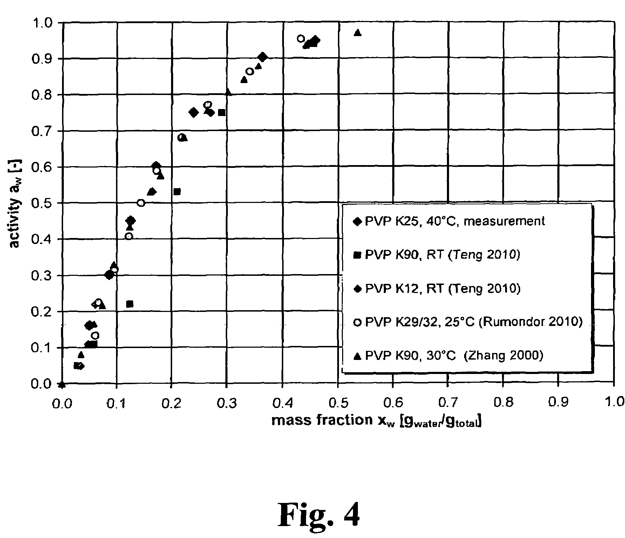

The measured values and literature values corresponded well for PVP, as shown in Figure 4, and the concentration dependence of the interaction parameter was found to fit the equation below. This equation was used to determine the amount of absorbed water by PVP in the mixtures.

$$\chi_{w.P} = 0.6954 - 0.1539 \cdot \varphi_w - 0.0392 \cdot \varphi_w^{-1}$$

EXAMPLE 3

Sodium chloride does not adsorb appreciable amount of water below the deliquescence point. Accordingly, the amount of water adsorbed below the deliquescence point at relative humidity of 75% is, at most, a few molecular layers. Above the deliquescence point, the sodium chloride crystals dissolve and the concentration of the solution is a function of the water activity in the gas phase and is calculated as:

$$a_w = \gamma_w \cdot (1 - x_s)$$

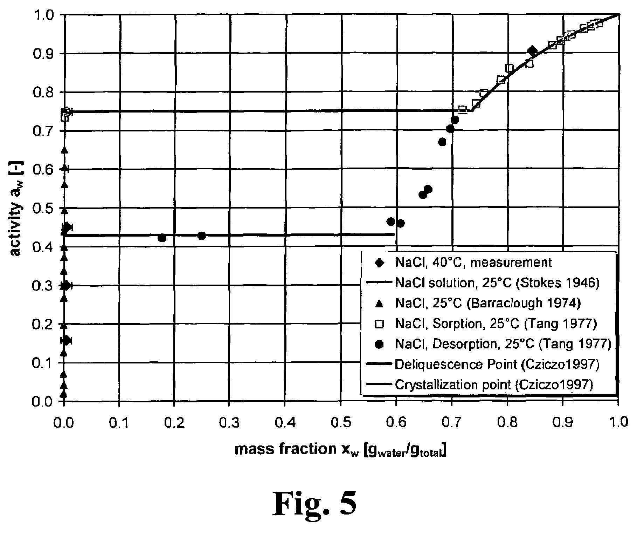

where $x_s$ is the molar fraction of salt in the solution and $\gamma_w$ is the activity coefficient. The activity coefficient was calculated from literature values and found to follow the following relationship to the water molar fraction (equation, Figure 5):

$$\gamma_w = -10.1757 \, x_w^2 + 20.9601 \, x_w + 9.7854$$

EXAMPLE 4

The sorption isotherms for PVP, NaCl, and mixtures of PVP and NaCl were compared. The results show that the mass of water adsorbed by the mixture is the weighted sums of the individual components. Below the deliquescence point of sodium chloride, the amount of water adsorbed by these mixtures decreases with the increasing amount of sodium chloride. However, the opposite is true at water activities above the deliquescence point of sodium chloride. Under these conditions, films with more sodium chloride have greater water sorption since sodium chloride adsorbs more water than PVP above the deliquescence point.

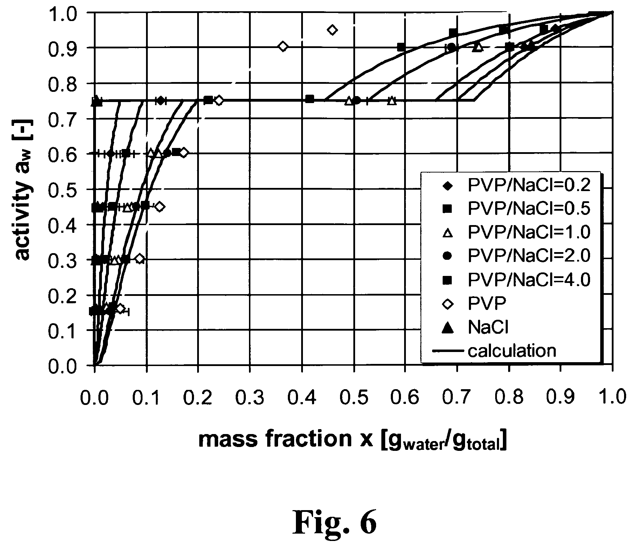

The comparison of individual components and mixtures is shown in Figure 6, where the PVP/NaCl ratio varied between 0.2 and 4.0. For the biosensors described above, the PVP/NaCl ratio ranged from 1.7 to 3.4. As shown in FIG. 6, the addition of NaCl to PVP causes the resulting matrix to adsorb less water. This is shown by the fact that the curve for PVP (alone) is to the relative right in the figure, and increasing the NaCl ratio from 0.5:1, to 1=1, to 2:1, to 5:1 pushes the curve progressively to the left. Accordingly, it has been shown by the inventors that the higher the concentration of salt in the matrix, the less water is adsorbed by the matrix.

EXAMPLE 5

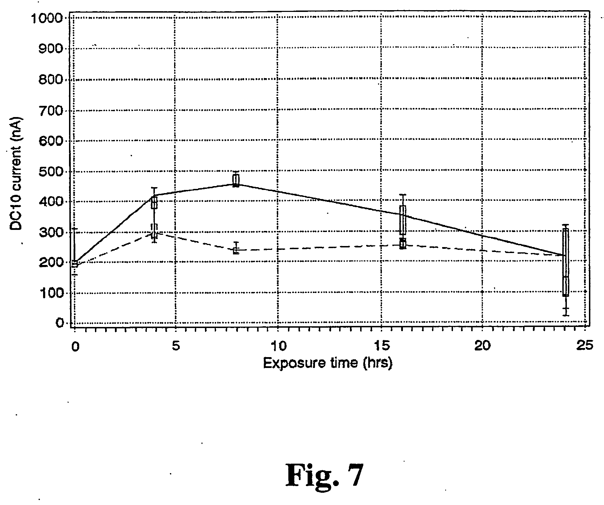

Deliquescent materials other than NaCl may also be used, as indicated above. For example, dry film reagents that are protected with a $MgSO_4$ deliquescent material have been shown to be effective using the procedures described generally above. In particular, FIG. 7 shows that including $MgSO_4$ in the dry film reagent matrix provides protection against water degradation in humid environments.

In FIG. 7, roll 2 shows data from a test of a dry film reagent comprising a FADGDH enzyme/cofactor system, a nitrosoaline mediator, PVP, and no deliquescent material. In contrast, roll 11 shows data from a test of a dry film reagent having a reagent mixture that is comparable to the reagent mixture of roll 2, but with about 6% $MgSO_4$ added as a deliquescent material. In these tests the temperature was about 30°C, and the relative humidity was about 82%.

Inspection of the data provided in FIG. 7 shows a significantly higher blank current from roll 2, where the dry film reagent matrix does not include a deliquescent material, than for roll 11, where the dry film reagent matrix does include a $MgSO_4$ deliquescent material. This indicates that including about 6% $MgSO_4$ in the dry film reagent mixture protects the reagent from degradation by water in the humid environment.

Additional work in the field of sorption isotherms is described in a paper titled "Sorption Isotherms of Mixtures of Polymers, Protein and Electrolytes - Measurement Data and Model Prediction," by Sibylle Kachel, Philip Scharfer, and Wilhelm Schabel of Thin Film Technology, Institute of Thermal Process Engineering, Karlsuhe Institute of Technology, Karlsruhe, Germany, the contents of which is hereby incorporated herein by reference.

Also, phase equilibrium data is reported in the following papers. D. J. Cziczo, J. B. Nowak, J. H. Hu, J. P. D. Abbatt, Infrared spectroscopy of model tropospheric aerosols as a function of relative humidity: Observation of deliquescence and crystallization, J. Geophys. Res. 102 (D15) (1997) 18843-18850.

P. B. Barraclough, P. G. Hall, The adsorption of water vapour by lithium fluoride, sodium fluoride and sodium chloride, Surf. Sci. 46 (2) (1974) 393-417.

I. Tang, H. Munkelwitz, J. Davis, Aerosol growth studies-ii. preparation and growth measurements of monodisperse salt aerosols, J. Aerosol Sci. 8 (3) (1977) 149-159.

R. H. Stokes, B. J. Levien, The osmotic and activity coefficients of zinc nitrate, zinc perchlorate and magnesium perchlorate. transference numbers in zinc perchlorate solutions, J. Am. Chem. Soc. 68 (2) (1946) 333-337.

J. Teng, S. Bates, D. A. Engers, K. Leach, P. Schields, Y. Yang, Effect of water vapor sorption on local structure of poly(vinylpyrrolidone), J. Pharm. Sci. 99 (9) (2010) 3815-3825.

J. Zhang, G. Zografi, The relationship between "BET" and "free volume"-derived parameters for water vapor absorption into amorphous solids, J. Pharm. Sci. 89 (8) (2000) 1063-1072.

A. C. F. Rumondor, H. Konno, P. J. Marsac, L. S. Taylor, Analysis of the moisture sorption behavior of amorphous drug-polymer blends, J. Appl. Polym. Sci. 117 (2) (2010) 1055-1063.

While certain preferred embodiments have been described in detail in the foregoing description, the same is to be considered as illustrative and not restrictive in character, it being understood that the preferred embodiment has been shown and described and that all changes, equivalents, and modifications that come within the scope of the inventions as defined by following claims are desired to be protected. It will be evident from the specification that aspects or features discussed in one context or embodiment will be applicable in other contexts or embodiments.

**Claims**

1. A test element for determining the amount of an analyte in a sample fluid, comprising:

   a) a working electrode and a counter electrode;
   b) a dry-film reagent matrix extending between the working electrode and the counter electrode and configured for an electrode reaction; wherein said reagent matrix comprises a deliquescent material and one or more active ingredients effective for facilitating a desired electrochemical reaction, and wherein the deliquescent material is selected and provided in an amount effective to preemptively absorb water from the atmosphere when the relative humidity exceeds a predetermined level, and wherein said deliquescent material has a deliquescence point between 50% relative humidity and 80% relative humidity, preferably between 70% relative humidity and 80% relative humidity, and more preferably at 75% relative humidity.

2. The test element of claim 1, wherein said deliquescent material comprises one or more members selected from the group consisting of sodium chloride, calcium chloride, magnesium chloride, magnesium sulfate, zinc chloride, potassium carbonate, potassium phosphate, carnallite, ferric ammonium citrate, potassium hydroxide and sodium hydroxide; and preferably comprises NaCl and/or $MgSO_4$.

3. The test element of claim 1 or 2, wherein said test element is a glucose meter test strip having a glucose reagent matrix thereon, and wherein said test strip reagent matrix includes an enzyme system and a mediator,

4. The test element of claim 3, wherein said enzyme system comprises glucose dehydrogenase (GDH) enzyme and/or flavin adenine dinucleotide (FAD) cofactor.

5. The test element of claim 3 or 4, wherein said mediator comprises a nitrosoaniline mediator.

6. The test element of any of the previous claims, wherein said reagent matrix includes a film former, said film former preferably comprising polyvinylpyrrolidone (PVP) and/or a PVP-containing material.

7. The test element of any of the previous claims, wherein said deliquescent material is provided in an amount effective to absorb water from the atmosphere at a rate that is faster than the rate at which the other components of the reagent matrix absorb water from the atmosphere, in particular when the relative humidity in the atmosphere exceeds 75%.

8. The test element of any of the previous claims, wherein said deliquescent material is selected and provided in an amount effective to preferentially absorb water from the atmosphere when compared to the water absorption properties of the other reagent components, thus protecting the other components of the reagent matrix from absorbing water from the atmosphere when the relative humidity exceeds the deliquescence point of the deliquescent material.

9. The test element of any of the previous claims, wherein said deliquescent material is provided in the reagent matrix in an amount effective to provide from 1% to 10% by weight percent, preferably at least 2% by weight percent, e.g. between 2% and 8% by weight percent, and more preferably at least 3%, e.g. between 3% and 7% by weight percent deliquescent material in the dry reagent matrix, based on the total weight of the dry reagent matrix.

10. The test element of any of the previous claims, wherein said deliquescent material is NaCl, and is provided in the reagent matrix in an amount effective to provide between 3.0% by weight percent and 6.0% by weight percent NaCl in the dry reagent matrix, based on the total weight of the dry reagent matrix.

11. The test element of any of the previous claims, wherein said deliquescent material is $MgSO_4$, and is provided in the reagent matrix in an amount effective to provide between 30% by weight percent and 7.0% by weight percent $MgSO_4$ in the dry reagent matrix, based on the total weight of the dry reagent matrix.

12. A method for improving the stability of a biosensor test element with respect to degradation of reagent components by humidity in the air, wherein the test element comprises a working electrode and a counter electrode, and a dry-film reagent matrix extending between the working electrode and the counter electrode and configured for an electrode reaction; the method comprising providing in the reagent matrix a deliquescent material selected and in an amount effective to decrease the sorption of water from the air by other components of the reagent matrix, and wherein said deliquescent material has a deliquescence point between 50% relative humidity and 80% relative humidity, preferably between 70% relative humidity and 80% relative humidity, and more preferably at 75% relative humidity.

**Patentansprüche**

1. Testelement zum Bestimmen der Menge eines Analyten in einer Flüssigprobe, das Folgendes umfasst:

   a) eine Arbeitselektrode und eine Gegenelektrode;
   b) eine Trockenfilm-Reagenzmatrix, die sich zwischen der Arbeitselektrode und der Gegenelektrode erstreckt und für eine Elektrodenreaktion ausgelegt ist; wobei die Reagenzmatrix ein unter Feuchtigkeitseinwirkung zerfließendes Material und einen oder mehrere Wirkbestandteile umfasst, die derart wirken, dass sie eine angestrebte elektrochemische Reaktion erleichtern; und wobei das unter Feuchtigkeitseinwirkung zerfließende Material derart ausgewählt und in einer derartigen Menge bereitgestellt wird, dass es auf vorbeugende Weise Wasser aus der Atmosphäre aufnimmt, wenn die relative Feuchtigkeit ein vorbestimmtes Ausmaß überschreitet, und wobei das unter Feuchtigkeitseinwirkung zerfließende Material einen Deliqueszenzpunkt hat, der zwischen 50 % relativer Feuchte und 80 % relativer Feuchte, vorzugweise zwischen 70 % relativer Feuchte und 80 % relativer Feuchte, und besonders bevorzugt bei 75 % relativer Feuchte liegt.

2. Testelement nach Anspruch 1, wobei das unter Feuchtigkeitseinwirkung zerfließende Material ein oder mehrere Mitglieder umfasst, die aus der Gruppe ausgewählt sind, welche aus Natriumchlorid, Calciumchlorid, Magnesiumchlorid, Magnesiumsulfat, Zinkchlorid, Kaliumcarbonat, Kaliumphosphat, Carnallit, Ammoniumeisen(III)-citrat, Kaliumhydroxid und Natriumhydroxid besteht; wobei es vorzugsweise NaCl und/oder $MgSO_4$ umfasst.

3. Testelement nach Anspruch 1 oder 2, wobei das Testelement ein Glucosemessstreifen ist, auf welchem sich eine Glucosereagenzmatrix befindet, und wobei die Teststreifen-Reagenzmatrix ein Enzymsystem und einen Mediator beinhaltet.

4. Testelement nach Anspruch 3, wobei das Enzymsystem Glucose-Dehydrogenase (GDH) als Enzym und/oder Flavin-Adenin-Dinukleotid (FAD) als Co-Faktor umfasst.

5. Testelement nach Anspruch 3 oder 4, wobei der Mediator einen Nitrosoanilin-Medioator umfasst.

6. Testelement nach einem der vorhergehenden Ansprüche, wobei die Reagenzmatrix einen Filmbildner umfasst, wobei der Filmbildner vorzugsweise Polyvinylpyrrolidon (PVP) und/oder ein PVP-haltiges Material umfasst.

7. Testelement nach einem der vorhergehenden Ansprüche, wobei das unter Feuchtigkeitseinwirkung zerfließende Material in einer derartigen Menge bereitgestellt wird, dass es Wasser aus der Atmosphäre mit einer Rate aufnimmt, die schneller als die Rate ist, mit welcher die übrigen Bestandteile der Reagenzmatrix Wasser aus der Atmosphäre aufnehmen, insbesondere wenn die relative Feuchtigkeit in der Atmosphäre 75 % übersteigt.

8. Testelement nach einem der vorhergehenden Ansprüche, wobei das unter Feuchtigkeitseinwirkung zerfließende Material derart ausgewählt wird und in einer derartigen Menge bereitgestellt wird, dass es bevorzugt Wasser aus der Atmosphäre aufnimmt, verglichen mit den Wasseraufnahmeeigenschaften der übrigen Reagenzbestandteile, um auf diese Weise die übrigen Bestandteile der Reagenzmatrix vor einer Wasseraufnahme aus der Atmosphäre zu schützen, wenn die relative Feuchtigkeit den Deliqueszenzpunkt des unter Feuchtigkeitseinwirkung zerfließenden Materials übersteigt.

9. Testelement nach einem der vorhergehenden Ansprüche, wobei das unter Feuchtigkeitseinwirkung zerfließende Material in der Reagenzmatrix in einer derartigen Menge bereitgestellt wird, dass 1 % bis 10 % in Gewichtsprozent, vorzugsweise mindestens 2 % nach Gewichtsprozent, z.B. zwischen 2 % und 8 % nach Gewichtsprozent, und besonders bevorzugt mindestens 3 %, z.B. zwischen 3 % und 7 % nach Gewichtsprozent, an unter Feuchtigkeitseinwirkung zerfließendem Material in der wasserfreien Reagenzmatrix bereitgestellt werden, bezogen auf das Gesamtgewicht der wasserfreien Reagenzmatrix.

10. Testelement nach einem der vorhergehenden Ansprüche, wobei es sich bei dem unter Feuchtigkeitseinwirkung zerfließenden Material um NaCl handelt und dieses in der Reagenzmatrix in einer derartigen Menge bereitgestellt wird, dass zwischen 3,0 % in Gewichtsprozent und 6,0 % in Gewichtsprozent an NaCl in der wasserfreien Reagenzmatrix bereitgestellt werden, bezogen auf das Gesamtgewicht der wasserfreien Reagenzmatrix.

11. Testelement nach einem der vorhergehenden Ansprüche, wobei es sich bei dem unter Feuchtigkeitseinwirkung zerfließenden Material um $MgSO_4$ handelt und es in der Reagenzmatrix in einer derartigen Menge bereitgestellt wird, dass zwischen 3,0 % in Gewichtsprozent und 7,0 % in Gewichtsprozent an $MgSO_4$ in der wasserfreien Reagenzmatrix bereitgestellt werden, bezogen auf das Gesamtgewicht der wasserfreien Reagenzmatrix.

**12.** Verfahren zur Verbesserung der Stabilität eines Biosensor-Testelements hinsichtlich einer Degradation von Reagenzbestandteilen durch Luftfeuchtigkeit, wobei das Testelement eine Arbeitselektrode und eine Gegenelektrode umfasst sowie eine Trockenfilm-Reagenzmatrix, die sich zwischen der Arbeitselektrode und der Gegenelektrode erstreckt und für eine Elektrodenreaktion ausgelegt ist; wobei das Verfahren das Bereitstellen, innerhalb der Reagenzmatrix, eines unter Feuchtigkeitseinwirkung zerfließenden Materials umfasst, welches derart ausgewählt ist und in einer derartigen Menge vorliegt, dass die Bindung von Wasser aus der Luft durch die übrigen Bestandteile der Reagenzmatrix verringert wird, und wobei das unter Feuchtigkeitseinwirkung zerfließende Material einen Deliqueszenzpunkt hat, der zwischen 50 % relativer Feuchtigkeit und 80 % relativer Feuchtigkeit, vorzugsweise zwischen 70 % relativer Feuchtigkeit und 80 % relativer Feuchtigkeit, und besonders bevorzugt bei 75 % relativer Feuchtigkeit liegt.

**Revendications**

**1.** Élément d'essai pour déterminer la quantité d'un analyte dans un liquide échantillon, comprenant :

a) une électrode de travail et une contre-électrode;
b) une matrice réactive pelliculée sèche s'étendant entre l'électrode de travail et la contre-électrode et configurée pour une réaction d'électrode; dans lequel ladite matrice réactive comprend un matériau déliquescent et une ou plusieurs matières actives efficaces pour faciliter une réaction électrochimique souhaitée; et dans lequel le matériau déliquescent est choisi et fourni en une quantité efficace pour absorber de façon préemptive l'eau de l'atmosphère lorsque l'humidité relative dépasse un niveau prédéterminé, et dans lequel ledit matériau déliquescent a un point de déliquescence compris entre 50 % d'humidité relative et 80 % d'humidité relative, de préférence entre 70 % d'humidité relative et 80 % d'humidité relative et plus préférablement égal à 75 % d'humidité relative.

**2.** Élément d'essai selon la revendication 1, dans lequel ledit matériau déliquescent comprend un ou plusieurs membres choisis dans le groupe constitué par le chlorure de sodium, le chlorure de calcium, le chlorure de magnésium, le sulfate de magnésium, le chlorure de zinc, le carbonate de potassium, le phosphate de potassium, la carnallite, le citrate d'ammonium ferrique, l'hydroxyde de potassium et l'hydroxyde de sodium; et comprend de préférence du NaCl et/ou du $MgSO_4$.

**3.** Élément d'essai selon la revendication 1 ou 2, ledit élément d'essai étant une bandelette réactive pour glucomètre sur laquelle est disposée une matrice réactive au glucose, et laquelle matrice réactive de la bandelette réactive comprend un système enzymatique et un médiateur.

**4.** Élément d'essai selon la revendication 3, dans lequel ledit système enzymatique comprend l'enzyme glucose déshydrogénase (GDH) et/ou le cofacteur flavine adénine dinucléotide (FAD).

**5.** Élément d'essai selon la revendication 3 ou 4, dans lequel ledit médiateur comprend un médiateur nitrosoaniline.

**6.** Élément d'essai selon l'une quelconque des revendications précédentes, dans lequel ladite matrice réactive comprend un filmogène, ledit filmogène comprenant de préférence une polyvinylpyrrolidone (PVP) et/ou un matériau contenant de la PVP.

**7.** Élément d'essai selon l'une quelconque des revendications précédentes, dans lequel ledit matériau déliquescent est fourni en une quantité efficace pour absorber l'eau de l'atmosphère à une vitesse plus rapide que la vitesse à laquelle les autres composants de la matrice réactive absorbent l'eau de l'atmosphère, en particulier lorsque l'humidité relative dans l'atmosphère dépasse 75 %.

**8.** Élément d'essai selon l'une quelconque des revendications précédentes, dans lequel ledit matériau déliquescent est choisi et fourni en une quantité efficace pour absorber de préférence l'eau de l'atmosphère par comparaison avec les propriétés d'absorption d'eau des autres composants réactifs, empêchant ainsi les autres composants de la matrice réactive d'absorber l'eau de l'atmosphère lorsque l'humidité relative dépasse le point de déliquescence du matériau déliquescent.

**9.** Élément d'essai selon l'une quelconque des revendications précédentes, dans lequel ledit matériau déliquescent est fourni dans la matrice réactive en une quantité efficace pour fournir entre 1 % et 10 % en poids, de préférence au moins 2 % en poids, par exemple entre 2 % et 8 % en poids, et plus préférablement au moins 3 %, par exemple entre 3 % et 7 % en poids de matériau déliquescent dans la matrice réactive sèche, sur la base du poids total de la matrice réactive sèche.

**10.** Élément d'essai selon l'une quelconque des revendications précédentes, dans lequel ledit matériau déliquescent est le NaCl, et est fourni dans la matrice réactive en une quantité efficace pour fournir entre 3,0 % et 6,0 % en poids de NaCl dans la matrice réactive sèche, sur la base du poids total de la matrice réactive sèche.

**11.** Élément d'essai selon l'une quelconque des revendications précédentes, dans lequel ledit matériau déliquescent est le $MgSO_4$, et est fourni dans la matrice réactive en une quantité efficace pour fournir entre 3,0 % et 7,0 % en poids de $MgSO_4$ dans la matrice réactive sèche, sur la base du poids total de la matrice réactive sèche.

**12.** Procédé d'amélioration de la stabilité d'un élément d'essai de biocapteur par rapport à la dégradation des composants réactifs par l'humidité dans l'air, dans lequel l'élément d'essai comprend une électrode de travail et une contre-électrode, et une matrice réactive pelliculée sèche s'étendant entre l'électrode de travail et la contre-électrode et configurée pour une réaction d'électrode ; le procédé comprenant la fourniture, dans la matrice réactive, d'un matériau déliquescent choisi et en une quantité efficace pour diminuer la sorption de l'eau de l'air par les autres composants de la matrice réactive, et dans lequel ledit matériau déliquescent a un point de déliquescence compris entre 50 % d'humidité relative et 80 % d'humidité relative, de préférence entre 70 % d'humidité relative et 80 % d'humidité relative et plus préférablement égal à 75 % d'humidité relative.

Fig. 1

Fig. 2

**Fig. 3**

**Fig. 4**

**Fig. 5**

**Fig. 6**

**Fig. 7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1174716 A
- US 20080031778 A
- WO 0250609 A
- WO 02083273 A
- US 5286362 A
- US 5128015 A

**Non-patent literature cited in the description**

- Sorption Isotherms of Mixtures of Polymers, Protein and Electrolytes - Measurement Data and Model Prediction. **SIBYLLE KACHEL ; PHILIP SCHARFER ; WILHELM SCHABEL.** Thin Film Technology. Institute of Thermal Process Engineering
- **D. J. CZICZO ; J. B. NOWAK ; J. H. HU ; J. P. D. ABBATT.** Infrared spectroscopy of model tropospheric aerosols as a function of relative humidity: Observation of deliquescence and crystallization. *J. Geophys. Res.,* 1997, vol. 102 (D15), 18843-18850
- **P. B. BARRACLOUGH ; P. G. HALL.** The adsorption of water vapour by lithium fluoride, sodium fluoride and sodium chloride. *Surf. Sci.,* 1974, vol. 46 (2), 393-417
- **I. TANG ; H. MUNKELWITZ ; J. DAVIS.** Aerosol growth studies-ii. preparation and growth measurements of monodisperse salt aerosols. *J. Aerosol Sci.,* 1977, vol. 8 (3), 149-159
- **R. H. STOKES ; B. J. LEVIEN.** The osmotic and activity coefficients of zinc nitrate, zinc perchlorate and magnesium perchlorate. transference numbers in zinc perchlorate solutions. *J. Am. Chem. Soc.,* 1946, vol. 68 (2), 333-337
- **J. TENG ; S. BATES ; D. A. ENGERS ; K. LEACH ; P. SCHIELDS ; Y. YANG.** Effect of water vapor sorption on local structure of poly(vinylpyrrolidone). *J. Pharm. Sci,* 2010, vol. 99 (9), 3815-3825
- **J. ZHANG ; G. ZOGRAFI.** The relationship between "BET" and "free volume"-derived parameters for water vapor absorption into amorphous solids. *J. Pharm. Sci.,* 2000, vol. 89 (8), 1063-1072
- **A. C. F. RUMONDOR ; H. KONNO ; P. J. MARSAC ; L. S. TAYLOR.** Analysis of the moisture sorption behavior of amorphous drug-polymer blends. *J. Appl. Polym. Sci.,* 2010, vol. 117 (2), 1055-1063